Europäisches Patentamt
European Patent Office
Office européen des brevets

(11) Veröffentlichungsnummer: **0 148 438**
**A2**

# EUROPÄISCHE PATENTANMELDUNG

(21) Anmeldenummer: **84115213.5**

(22) Anmeldetag: **12.12.84**

(51) Int. Cl.⁴: **C 07 D 487/04**, A 61 K 31/495, A 61 K 31/50, A 61 K 31/415 // (C07D487/04, 237:00, 235:00),(C07D487/04, 241:00, 235:00),(C07D487/04, 253:00, 235:00)

(30) Priorität: **23.12.83 DE 3346640**

(43) Veröffentlichungstag der Anmeldung: **17.07.85 Patentblatt 85/29**

(84) Benannte Vertragsstaaten: **AT BE CH DE FR GB IT LI LU NL SE**

(71) Anmelder: **Dr. Karl Thomae GmbH, Postfach 1755, D-7950 Biberach (Riss) (DE)**

(72) Erfinder: **Austel, Volkhard, Dr. Dipl.-Chem., Kapellenweg 7, D-7950 Biberach 1 (DE)**
Erfinder: **Hauel, Norbert, Dr. Dipl.-Chem., Händelstrasse 12, D-7950 Biberach 1 (DE)**
Erfinder: **Heider, Joachim, Dr. Dipl.-Chem., Am Hang 3, D-7951 Warthausen 1 (DE)**
Erfinder: **Reiffen, Manfred, Dr. Dipl.-Chem., Matthias-Erzberger-Strasse 40, D-7950 Biberach 1 (DE)**
Erfinder: **van Meel, Jacobus Constantinus A., Dr. Pharm., Amriswilstrasse 7, D-7950 Biberach 1 (DE)**
Erfinder: **Diederen, Willi, Dr. Pharm., Haldenstrasse 1a, D-7950 Biberach 1 (DE)**

(54) **Neue Imidazolderivate, deren Herstellung und diese Verbindungen enthaltende Arzneimittel.**

(57) Die Erfindung betrifft neue Imidazolderivate der allgemeinen Formel

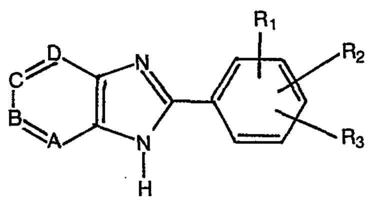

(I),

in der die Reste A bis D und $R_1$ bis $R_3$ wie im Anspruch 1 definiert sind, deren Tautomere und deren Säureadditionssalze, insbesondere deren physiologisch verträgliche Säureadditionssalze mit anorganischen oder organischen Säuren, welche wertvolle pharmakologische Eigenschaften aufweisen, insbesondere eine Wirkung auf die Kontraktilität des Herzmuskels.

Die neuen Verbindungen lassen sich nach für analoge Verbindungen bekannten Verfahren herstellen.

DR. KARL THOMAE GMBH
D-7950 Biberach 1

Case 5/894
Dr.Fl./Kp.

# Neue Imidazolderivate, deren Herstellung und diese Verbindungen enthaltende Arzneimittel

In der EP-A-0.024.290 werden bereits 2-Phenyl-imidazolderivate beschrieben, welche wertvolle pharmakologische Eigenschaften aufweisen.

Es wurde nun gefunden, daß die neuen Imidazolderivate der allgemeinen Formel

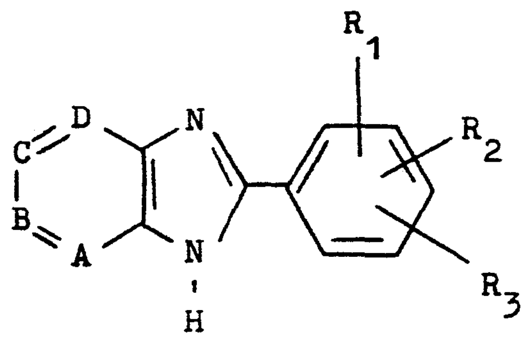

, (I)

deren Tautomere und deren Säureadditionssalze, insbesondere deren physiologisch verträgliche Säureadditionssalze mit anorganischen oder organischen Säuren, welche sich von den bekannten Verbindungen durch die Reste A, B, C und D unterscheiden, überlegene pharmakologische Eigenschaften aufweisen, insbesondere eine Wirkung auf die Kontraktilität des Herzmuskels.

In der obigen allgemeinen Formel I bedeutet

jeder der Reste A bis D ein N-Atom oder eine CH-Gruppe, wobei jedoch mindestens 2 der Reste A bis D N-Atome darstellen müssen und mit der Maßgabe, daß, wenn A und C gleichzeitig N-Atome bedeuten, auch B und/oder D je ein N-Atom darstellen muß,

$R_1$ ein Halogenatom, eine Alkyl-, Cyan-, Amino-, Alkylamino-, Benzyloxy-, Aminocarbonyl-, Alkylaminocarbonyl-Dialkylaminocarbonyl-, Carboxy-, Alkoxycarbonyl-, Alkylsulfonyloxy-, Alkylsulfonyl-, Aminosulfonyl-, Alkylaminosulfonyl-, Dialkylaminosulfonyl-, Nitro-, Alkanoylamino-, Alkylsulfonylamino- oder N-Alkyl-alkylsulfonylaminogruppe oder auch, wenn 3 oder 4 der Reste A bis D je ein N-Atom und 0 oder 1 der Reste A bis D eine CH-Gruppe oder A und B je ein N-Atom und C und D je eine CH-Gruppe darstellen,
ein Wasserstoffatom, eine Hydroxy-, Alkoxy-, Phenylalkoxy-, Alkylmercapto-, Alkylsulfinyl-, Dialkylamino-, Cyanalkoxy-, Alkenyloxy- oder Alkinyloxygruppe,

$R_2$ und $R_3$, die gleich oder verschieden sein können, Wasserstoff- oder Halogenatome, Alkyl-, Hydroxy-, Alkoxy-, Phenylalkoxy-, Alkylmercapto-, Alkylsulfinyl-, Alkylsulfonyl-, Aminosulfonyl-, Alkylaminosulfonyl-, Dialkylaminosulfonyl-, Alkylsulfonyloxy-, Alkylsulfonylamino-, N-Alkyl-alkylsulfonylamino-, Nitro-, Amino-, Alkylamino-, Dialkylamino-, Cyanalkoxy-, Cyan-, Carboxy-, Alkoxycarbonyl-, Aminocarbonyl-, Alkylaminocarbonyl-, Dialkylaminocarbonyl-, N-Alkanoyl-amino-, Alkenyloxy- oder Alkinyloxygruppe, wobei der Alkylteil jeweils 1 bis 3 Kohlenstoffatome und der Alkenyl- bzw. Alkinylteil jeweils 3 bis 5 Kohlenstoffatome enthalten kann.

Gegenstand der vorliegenden Erfindung sind somit die neuen Imidazo[4,5-b]pyrazine, Imidazo[4,5-c]pyridazine, Imidazo-[4,5-d]pyridazine, Imidazo[4,5-d]-1,2,3-triazine, Imidazo-[4,5-e]-1,2,4-triazine und Imidazo[4,5-e]tetrazine der obigen allgemeinen Formel I, deren Tautomere, deren Säureadditionssalze, insbesondere deren physiologisch verträgliche Säureadditionssalze mit anorganischen oder organischen Säuren, und diese Verbindungen enthaltende Arzneimittel sowie Verfahren zu ihrer Herstellung.

Für die bei der Definition der Reste $R_1$, $R_2$ und $R_3$ eingangs erwähnten Bedeutungen kommt beispielsweise für $R_1$, $R_2$ und/oder $R_3$ jeweils die des Wasserstoff-, Fluor-, Chlor- oder Bromatoms, der Methyl-, Ethyl-, n-Propyl-, Isopropyl-, Hydroxy-, Methoxy-, Ethoxy-, n-Propoxy-, Isopropoxy-, Benzyloxy-, 1-Phenylethoxy-, 1-Phenyl-n-propoxy-, 1-Methyl-1-phenyl-ethoxy-, 2-Phenyl-ethoxy-, 3-Phenyl-n-propoxy-, Methylmercapto-, Ethylmercapto-, n-Propylmercapto-, Methylsulfinyl-, Ethylsulfinyl-, Isopropylsulfinyl-, Methylsulfonyl-, Ethylsulfonyl-, n-Propylsulfonyl-, Aminosulfonyl-, Methylaminosulfonyl-, Ethylaminosulfonyl-, n-Propylaminosulfonyl-, Dimethylaminosulfonyl-, Diethylaminosulfonyl-, Diisopropylaminosulfonyl-, Ethyl-methylaminosulfonyl-, Methylsulfonyloxy-, Ethylsulfonyloxy-, n-Propylsulfonyloxy-, Methylsulfonylamino-, Ethylsulfonylamino-, Isopropylsulfonylamino-, N-Methyl-methylsulfonylamino-, N-Ethyl-methylsulfonylamino-, N-Methyl-ethylsulfonylamino-, N-n-Propyl-ethylsulfonylamino-, N-Methyl-n-propylsulfonylamino-, N-Ethyl-isopropylaminosulfonyl-, Amino-, Methylamino-, Ethylamino-, n-Propylamino-, Isopropylamino-, Dimethylamino-, Diethylamino-, Di-n-propylamino-, N-Ethylmethylamino-, N-Methyl-isopropylamino-, Cyanmethoxy-, 1-Cyanethoxy-, 2-Cyanethoxy-, 3-Cyanpropoxy-, Cyan-, Nitro-, Carboxy-, Methoxycarbonyl-, Ethoxycarbonyl-, n-Propoxycarbonyl-, Isopropoxycarbonyl-, Aminocarbonyl-, Methylaminocarbo-

nyl-, Ethylaminocarbonyl-, Isopropylamino- carbonyl-, Dimethylaminocarbonyl-, Diethylaminocarbonyl-, Di-n-propylaminocarbonyl-, N-Ethyl-methylaminocarbonyl-, N-Ethyl-n-propylaminocarbonyl-, N-Formylamino-, N-Acetyl- amino-, N-Propionylamino-, Allyloxy-, But-2-en-yloxy-, Pent-2-enyloxy-, Propargyloxy-, But-2-inyloxyoder Pent-2- inyloxygruppe in Betracht.

Bevorzugte Verbindungen der obigen allgemeinen Formel I sind diejenigen, in denen
3 oder 2 der Reste A bis D N-Atome und die übrigen der Reste A bis D CH-Gruppen darstellen, mit der Maßgabe, wenn A und C gleichzeitig N-Atome bedeuten, auch einer der Reste B und D ein N-Atom darstellt,

$R_1$ und $R_2$, ein Halogenatom, eine Methyl-, Methoxy-, Benzyloxy-, Ethoxy-, Propoxy-, Dimethylamino-, Amino-, Aminocarbonyl-, Aminosulfonyl-, Methylaminosulfonyl-, Dimethylaminosulfonyl-, Cyan-, Nitro-, Methylmercapto-, Methylsulfinyl-, Methylsulfonyl-, Methansulfonyloxy-, Methansulfonylamino-, N-Methyl-methansulfonylamino-, Propargyloxy- oder Cyanmethoxygruppe und

$R_3$ ein Wasserstoffatom, eine Dimethylamino-, Methoxy-, Ethoxy-, Propoxy- oder Methylmercaptogruppe bedeuten, deren Tautomere und deren Säureadditionssalze, insbesondere deren physiologisch verträgliche Säureadditionssalze.

Erfindungsgemäß erhält man die neuen Verbindungen nach folgenden Verfahren:

a) Cyclisierung einer gegebenenfalls im Reaktionsgemisch hergestellten Verbindung der allgemeinen Formel

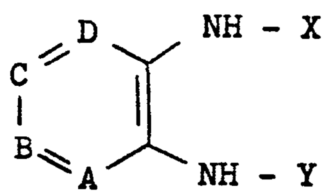

,(II)

in der A bis D wie eingangs definiert sind, einer der Reste X oder Y ein Wasserstoffatom und der andere der beiden Reste X und Y oder beide Reste X und Y eine Gruppe der Formel

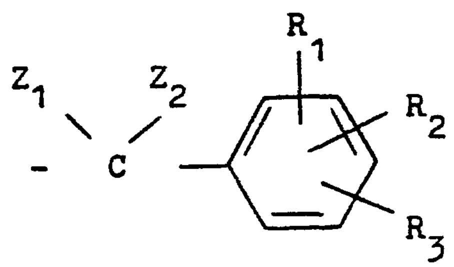

darstellen, in der

$R_1$ bis $R_3$ wie eingangs definiert sind, $Z_1$ und $Z_2$, die gleich oder verschieden sein können, Halogenatome, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen mono- oder disubstituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester, Amid oder Methojodid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Salzsäure, Phosphorsäure, Polyphosphorsäure, Essigsäureanhydrid oder gegebenenfalls auch in Gegenwart einer Base wie Kaliumäthylat oder Kalium-tert.butylat durchgeführt. Die Cyclisierung kann jedoch auch ohne Lösungsmittel und/oder Kondensationsmittel durchgeführt werden.

b) Zur Herstellung von Verbindungen der allgemeinen Formel I, in der mindestens einer der Reste $R_1$, $R_2$ oder $R_3$ eine Alkylsulfinyl- oder Alkylsulfonylgruppe darstellt:

Oxidation einer Verbindung der allgemeinen Formel

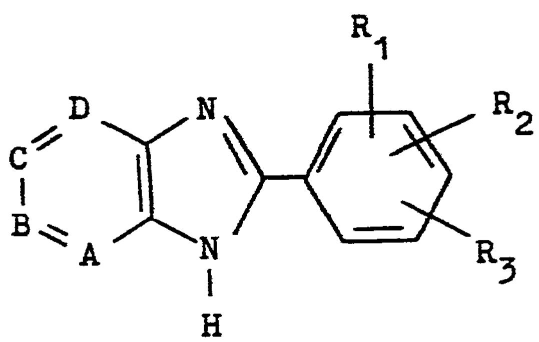

,(III)

in der
A bis D und $R_1$ bei $R_3$ wie eingangs definiert sind, wobei jedoch mindestens einer der Reste $R_1$, $R_2$ oder $R_3$ eine Alkylmercapto- oder Alkylsulfinylgruppe mit jeweils 1 bis 3 Kohlenstoffatomen darstellen muß.

Die Oxidation wird vorzugsweise in einem Lösungsmittel oder Lösungsmittelgemisch, z.B. in Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer Alkylsulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wässrigem Methanol oder Äthanol bei -15 bis 25°C, mit Brom in Eisessig oder wässriger Essigsäure gegebenenfalls in Gegenwart einer Base wie Natriumacetat, mit N-Brom-succinimid in Äthanol, mit tert.Butyl-hyprochlorit in Methanol bei -80 bis -30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmäßigerweise mit wäßrigem Äthanol hydrolysiert.

Zur Herstellung einer Alkylsulfonylverbindung der allgemeinen Formel I wird die Oxidation zweckmäßigerweise mit einem bzw. mit zwei oder mehr Äquivalenten des verwendeten Oxidationsmittels durchgeführt, z.B. mit Wasserstoffperoxid in Eisessig, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

c) Zur Herstellung von Verbindungen der allgemeinen Formel I, in der mindestens einer der Reste $R_1$, $R_2$ oder $R_3$ eine Alkylsulfonyloxy-, Alkylsulfonylamino-, N-Alkylalkylsulfonylamino- oder Alkanoylaminogruppe darstellt:

Umsetzung einer Verbindung der allgemeinen Formel

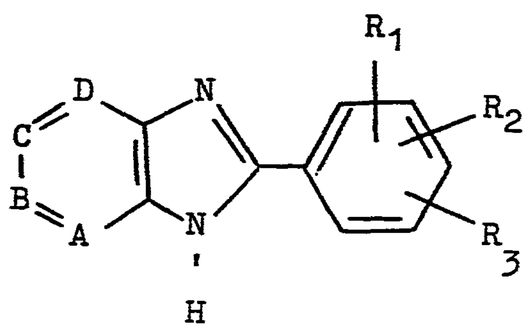

,(IV)

in der

A bis D und $R_1$ bis $R_3$ wie eingangs definiert sind, wobei jedoch mindestens einer der Reste $R_1$, $R_2$ oder $R_3$ eine Hydroxy-, Amino- oder N-Alkylaminogruppe mit 1 bis 3 Kohlenstoffatomen im Alkylteil darstellen muß, mit einer Säure der allgemeinen Formel

$$R_4 - WOH \qquad ,(V)$$

in der

$R_4$ eine Alkylgruppe mit 1 bis 3 Kohlenstoffatomen und

W eine Sulfonyl- oder Carbonylgruppe darstellen, in Gegenwart eines wasserentziehenden und/oder die Säure oder das Amin aktivierenden Mittels oder mit deren reaktionsfähigen Derivaten.

Die Umsetzung wird zweckmäßigerweise in einem Lösungsmittel oder Lösungsmittelgemisch wie Methylenchlorid, Ether, Tetrahydrofuran, Dioxan, Wasser oder Benzol gegebenenfalls in Gegenwart eines säurebindenden Mittels wie Natriumcarbonat, Natriumhydroxid, Triethylamin oder Pyridin, wobei die beiden letzteren gleichzeitig auch als Lösungsmittel verwendet werden können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der allgemeinen Formel V, z.B. mit deren Anhydrid oder Halogenid wie Methansulfonsäurechlorid oder Ethansulfonsäurechlorid, vorzugsweise bei Temperaturen zwischen 0 und 100°C, z.B. bei Temperaturen zwischen Raumtemperatur und 50°C, durchgeführt.

d) Zur Herstellung von Verbindungen der allgemeinen Formel I, in der mindestens einer der Reste $R_1$, $R_2$ oder $R_3$ eine durch eine Amino-, Alkylamino- oder Dialkylaminogruppe substituierte Carbonyl- oder Sulfonylgruppe darstellt:

Umsetzung einer Verbindung der allgemeinen Formel

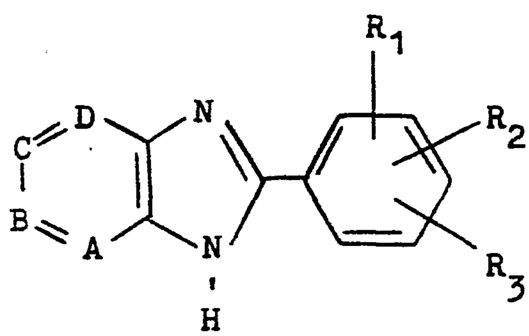

,(VI)

in der A bis D und $R_1$ bis $R_3$ wie eingangs definiert sind, wobei jedoch mindestens einer der Reste $R_1$, $R_2$ oder $R_3$ eine Carboxyl- oder Hydroxysulfonylgruppe darstellen muß, oder eines reaktionsfähigen Derivates hiervon mit einem Amin der allgemeinen Formel

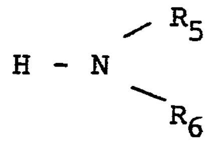

(VII)

in der

$R_5$ und $R_6$, die gleich oder verschieden sein können, Wasserstoffatome oder Alkylgruppen mit 1 bis 3 Kohlenstoffatome darstellen, oder mit einem reaktionsfähigen Derivat hiervon, falls mindestens einer der Reste $R_1$, $R_2$ oder $R_3$ die Carboxyl- oder Hydroxysulfonylgruppe darstellt.

Die Umsetzung wird zweckmäßigerweise in einem Lösungsmittel oder Lösungsmittelgemisch wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phorphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzung in einem entsprechenden Halogenid, z.B. dem Carbonsäure- oder Sulfonsäurechlorid, und einem entsprechenden Amin, wobei dieses gleichzeitig als Lösungsmittel dienen kann, und bei Temperaturen zwischen 0 und 50°C durchgeführt.

e) Zur Herstellung von Verbindungen der allgemeinen Formel I , in der mindestens einer der Reste $R_1$, $R_2$ oder $R_3$ eine Cyanalkoxy-, Alkenyloxy- oder Alkinyloxygruppe oder $R_2$ und/oder $R_3$ eine Alkoxygruppe darstellen:

Umsetzung einer Verbindung der allgemeinen Formel

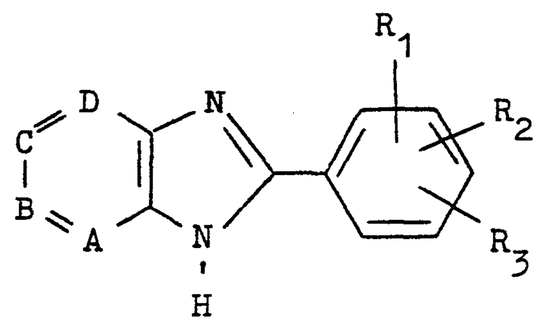

,(VIII)

in der
A bis D und $R_1$ bis $R_3$ wie eingangs definiert sind, wobei jedoch mindestes einer der Reste $R_1$, $R_2$ oder $R_3$ eine Hydroxygruppe darstellen muß, mit einer Verbindung der allgemeinen Formel

$$R_7 - U \quad ,(IX)$$

in der
$R_7$ eine gegebenenfalls durch eine Cyangruppe substituierte Alkylgruppe mit 1 bis 3 Kohlenstoffatomen, eine Alkenyl- oder Alkinylgruppe mit jeweils 3 bis 5 Kohlenstoffatomen und
U eine nukleophile Austrittsgruppe wie ein Halogenatom, eine substituierte Sulfonyloxygruppe oder eine Alkoxysulfonyloxygruppe bedeuten.

Die Umsetzung wird mit einem entsprechenden Alkylierungsmittel wie Methyljodid, Ethyljodid, Diethylsulfat, Dimethylsulfat, Chloracetonitril, Allylbromid oder Propargylbromid zweckmäßigerweise in einem Lösungsmittel oder Lösungsmittelgemisch wie Methylenchlorid, Ether, Tetrahydrofuran, Dioxan, Wasser oder Benzol gegebenenfalls in Gegenwart eines säurebindenden Mittels wie Natriumcarbonat, Natriumhydroxid, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Erhält man erfindungsgemäß eine Verbindung der allgemeinen Formel I, in der einer der Reste $R_1$, $R_2$ oder $R_3$ eine Benzyloxygruppe darstellt, so kann diese mittels Entbenzylierung in die entsprechende Hydroxyverbindung übergeführt werden, und/oder

eine erhaltene Verbindung der allgemeinen Formel I, in der mindestens einer der Reste $R_1$, $R_2$ oder $R_3$ eine Cyangruppe darstellt, so kann diese mittels Hydrolyse in die entsprechende Aminocarbonylverbindung übergeführt werden, und/oder

eine erhaltene Verbindung der allgemeinen Formel I, in der mindestens einer der Reste $R_2$ oder $R_3$ eine Nitrogruppe darstellt, so kann diese mittels Reduktion in die entsprechende Aminoverbindung übergeführt werden, und/oder

eine erhaltene Verbindung der allgemeinen Formel I, in der einer der Reste $R_1$, $R_2$ oder $R_3$ eine Aminogruppe darstellt, so kann diese über ihr Diazoniumsalz in eine entsprechende Verbindung der allgemeinen Formel I, in der einer der Reste $R_1$, $R_2$ oder $R_3$ ein Halogenatom, eine Hydroxy- oder Cyangruppe darstellt, übergeführt werden.

Die nachträgliche Entbenzylierung wird vorzugsweise in einem Lösungsmittel wie Wasser, Wasser/Ethanol, Methanol, Eisessig, Essigsäureethylester oder Dimethylformamid zweckmäßigerweise mit Wassertoff in Gegenwart eines Hydrierungskatalysators wie Raney-Nickel, Platin oder Palladium/Kohle bei Temperaturen zwischen 0 und 50°C, vorzugsweise jedoch bei Raumtemperatur, durchgeführt.

Die nachträgliche Hydrolyse wird in Gegenwart einer anorganischen Base mit Wasserstoffperoxid, z.B. mit 2 n Natronlauge oder Kalilauge/Waserstoffperoxid, bei Temperaturen zwischen 0 und 50°C, vorzugsugsweise jedoch bei Raumtemperatur, durchgeführt.

Die nachträgliche Reduktion der Nitroverbindung wird vorzugsweise in einem Lösungsmittel wie Wasser, Wasser/Äthanol, Methanol, Eisessig, Essigsäureäthylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)-chlorid oder Natriumdithionit oder mit Hydrazin in Gegenwart von Raney-Nickel bei Temperaturen zwischen 0 und 50°C, vorzugsweise jedoch bei Raumtemperatur, durchgeführt.

Die nachträgliche Umsetzung eines Diazoniumsalzes, z.B. des Fluoroborats, des Fluorides in 40%iger Flußsäure, des Hydrosulfats in Schwefelsäure oder des Hydrochlorids, erforderlichenfalls in Gegenwart von Kupfer oder eines entsprechenden Kupfer-(I)-Salzes wie Kupfer-(I)-chlorid/Salzsäure oder Kupfer-(I)-bromid/Bromwasserstoffsäure, wird bei leicht erhöhten Temperaturen, z.B. bei Temperaturen zwischen 15°C und 100°C, durchgeführt. Das erforderliche Diazoniumsalz wird zweckmäßigerweise in einem geeigneten Lösungsmittel, z.B. in Wasser/Salzsäure, Methanol/Salzsäure, Äthanol/Salzsäure oder Dioxan/Salzsäure, durch Diazotierung einer ent-

sprechenden Aminoverbindung mit einem Nitrit, z.B. Natriumnitrit oder einem Ester der salpetrigen Säure, bei niedrigen Temperaturen, z.B. bei Temperaturen zwischen -10°C und 5°C, hergestellt.

Die erhaltenen Verbindungen der allgemeinen Formel I können anschließend gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Furmarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis IX sind teilweise literaturbekannt bzw. erhält man sie nach literaturbekannten Verfahren. So erhält man beispielsweise die als Ausgangsstoffe verwendeten Verbindungen der allgemeinen Formel II durch Acylierung der entsprechenden o-Diaminoverbindungen bzw. durch Reduktion der entsprechenden Acylamino-nitro-Verbindungen sowie die Verbindungen der allgemeinen Formeln III, IV, VI oder VIII durch anschließenden Ringschluß (siehe BE-PS 810.545 und EP-A-0.024.290) und gegebenenfalls anschließende Entbenzylierung.

Wie bereits eingangs erwähnt weisen die neuen Verbindungen der allgemeinen Formel I, deren Tautomere und deren physiologisch verträgliche Säureadditionssalze überlegene pharmakologische Eigenschaften auf, insbesondere eine blutdrucksenkende und/oder positiv inotrope Wirkung.

Beispielweise wurden die Verbindungen

A = 2-(2-Methoxy-4-methansulfonyloxy-phenyl)-imidazo[4,5-b]-pyrazin und

B = 2-(2-Methoxy-4-methansulfonyloxy-phenyl)-imidazo[4,5-d]-pyridazin

auf ihre biologischen Eigenschaften wie folgt untersucht:

Bestimmung der Blutdruckwirkung und der positiv inotropen Wirkung an der narkotisierten Katze

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparamter $dp/dt_{max}$ mittels eines Analog-differenzierers gewonnen. Die zu untersuchenden Substanzen wurden in eine Vena femoralis injiziert. Als Lösungsmittel diente Polydiol 200. Jede Substanz wurde an mindestens 3 Katzen geprüft.

Die nachfolgende Tabelle enthält die Mittelwerte:

| Substanz | Dosis mg/kg | Zunahme von $dp/dt_{max}$ in % | Blutdruckwirkung in mm Hg | Wirkungsdauer (Halbwertszeit) in Minuten |
|---|---|---|---|---|
| A | 0,6 | 54 | -24/-21 | 20 |
| B | 0,3 | 36 | -31/-18 | 38 |

Die neuen Verbindungen sind gut verträglich, so konnten bei der Untersuchung der Substanz A keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern bzw. durch zusätzliche Blutdrucksenkung die Entleerung des Herzens erleichtern.

Hierzu lassen sich die neuen Verbindungen sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,1 - 5 mg/kg Körpergewicht, vorzugsweise jedoch 0,5 - 2,0 mg/kg Körpergewicht.

Die nachfolgenden Beispiele sollen die Erfindung näher erläutern:

Beispiel 1

2-(2,4-Dimethoxy-phenyl)-imidazo[4,5-c]pyridazin-hydrochlorid

2,7 g 3,4-Diamino-pyridazin-hydrochlorid werden 0,5 Stunden in 10 ml Triethylamin gekocht. Überschüssiges Triethylamin wird abgezogen, der Rückstand in 15 ml Glycol gelöst und nach Zugabe von 4,1 g S-Methyl-2,4-dimethoxy-thiobenzoesäure-morpholid-jodid 2 Stunden auf 120°C erhitzt. Das entstandene Methylmercaptan wird im Vakuum entfernt, die verbleibende Lösung mit Wasser versetzt und mehrfach mit Chloroform extrahiert. Die Chloroformphase wird eingedampft und der Rückstand durch Chromatographie auf Kieselgel (Elutionsmittel: Chloroform/Methanol = 100:0 bis 100:3) gereinigt. Durch Auflösen in wenig 2n Salzsäure und Versetzen mit konzentrierter Salzsäure wird das Hydrochlorid gefällt.
Ausbeute: 0,21 g (7 % der Theorie),
Schmelzpunkt: 245-246°C (Zersetzung).

Beispiel 2

2-(2-Methoxy-4-benzyloxy-phenyl)-imidazo[4,5-b]pyrazin

Eine Mischung aus 2,2 g 2,3-Diaminopyrazin und 5,16 g 2-Methoxy-4-benzyloxy-benzoesäure wird in einer Reibschale fein verrieben und dann in 80 ml Phosphoroxychlorid suspendiert. Man erhitzt unter Rühren 1,5 Stunden zum Rückfluß. Nach Abkühlen wird das Reaktionsgemisch mit Eiswasser zersetzt. Die erhaltene Lösung wird mit Ammoniak neutralisiert und mit Essigester extrahiert. Die Essigesterphasen werden eingedampft und der Rückstand mit Aceton verrieben, wobei das Produkt in reiner Form zurückbleibt. Eine zweite Fraktion wird durch Eindampfen der Acetonphase und Reinigung des Rückstandes über Kieselgel (Elutionsmittel: Methylenchlorid/ Ethanol = 100:0 bis 100:4) erhalten.
Ausbeute: 3,2 g (48 % der Theorie),
Schmelzpunkt: 178-180°C.

Beispiel 3

2-(2-Methoxy-4-methansulfonyloxy-phenyl)-imidazo[4,5-b]pyrazin

a) 2-(2-Methoxy-4-hydroxy-phenyl)-imidazo[4,5-b]pyrazin

3,15 g 2-(2-Methoxy-4-benzyloxy-phenyl-imidazo[4,5-b]pyrazin werden in 100 ml Methanol gelöst und 2,6 g 10%ige Palladiumkohle zugesetzt. Man hydriert 15 Stunden mit Wasserstoff bei 5 bar und Raumtemperatur. Das ausgefallene Produkt wird zusammen mit dem Katalysator abgesaugt. Der feste Rückstand wird mit einem Gemisch aus gleichen Teilen Wasser und Dimethylformamid extrahiert. Die Extrakte werden mit der obigen Mutterlauge vereinigt, eingeengt und der kristalline Rückstand mit Ether/Aceton verrieben und abgesaugt.
Ausbeute: 1,45 g (63 % der Theorie),
Schmelzpunkt: über 250°C.

b) 2-(2-Methoxy-4-methansulfonyloxy-phenyl)-imidazo[4,5-b]-pyrazin

1,4 g 2-(2-Methoxy-4-hydroxy-phenyl)-imidazo[4,5-b]pyrazin werden in 40 ml Pyridin suspendiert. Zu der Mischung werden 1,4 g Methansulfonylchlorid, gelöst in 10 ml Pyridin, zugetropft. Man erwärmt kurzzeitig auf 40°C und rührt dann über Nacht bei Raumtemperatur weiter. Das Reaktionsgemisch wird auf Wasser gegossen und mit Essigester extrahiert. Die

Essigesterphasen werden eingeengt und der Rückstand mit 2n Salzsäure verrührt. Das erhaltene Festprodukt wird aus 95 ml äthanolischer Salzsäure + 5 ml Wasser umkristallisiert.
Ausbeute: 1,25 g (68 % der Theorie),
Schmelzpunkt: 207-209°C.

Beispiel 4

2-(2,4-Dimethoxy-5-aminosulfonyl-phenyl)-imidazo[4,5-b]pyrazin

a) 2-(2,4-Dimethoxy-5-chlorsulfonyl-phenyl)-imidazo[4,5-b]-pyrazin

Hergestellt analog Beispiel 2 aus 2,3-Diamino-pyrazin und 2,4-Dimethoxy-5-chlorsulfonyl-benzoylchlorid.
Schmelzpunkt: 294-296°C.

b) 2-(2,4-Dimethoxy-5-aminosulfonyl-phenyl)-imidazo[4,5-b]-pyrazin

1,4 g 2-(2,4-Dimethoxy-5-chlorsulfonyl-phenyl)-imidazo-[4,5-b]-pyrazin werden eine Stunde mit 25 ml konzentriertem Ammoniak verrührt. Der gebildete Niederschlag wird mit einer Mischung aus gleichen Teilen Aceton und Ethanol ausgekocht.
Ausbeute: 0,95 g (71 % der Theorie),
Schmelzpunkt: 250-252°C

Beispiel 5

2-(2-Methoxy-4-methylsulfonyl-phenyl)-imidazo[4,5-b]pyrazin

0,7 g 2-(2-Methoxy-4-methylmercapto-phenyl)-imidazo[4,5-b]-pyrazin werden in 28 ml 90%iger Essigsäure suspendiert und

mit 0,45 ml 30%igem Wasserstoffperoxid versetzt. Man fügt nun solange Salzsäure zu bis eine klare Lösung entstanden ist. Man läßt über Nacht stehen und saugt den entstandenen Niederschlag ab. Er wird mit methanolischer Salzsäure aufgekocht, abgesaugt und mit Aceton und Ether gewaschen.
Ausbeute: 0,42 g (58 % der Theorie),
Schmelzpunkt: 273-276°C.

Beispiel 6

2-(2-Methoxy-4-methyl-phenyl)-imidazo[4,5-d]pyridazin-hydrochlorid

Hergestellt analog Beispiel 1 aus 4,5-Diamino-pyridazin und S-Methyl-2-methoxy-4-methyl-thiobenzoesäure-morpholid.
Schmelzpunkt: 216-218°C (Zers.).

Beispiel 7

2-(2-Methoxy-4-methansulfonylamino-phenyl)-imidazo[4,5-b]-pyrazin

Hergestellt analog Beispiel 2 aus 2,3-Diamino-pyrazin und 2-Methoxy-4-methansulfonylamino-benzoesäure.
Schmelzpunkt: 263-265°C.

Beispiel 8

2-(2-Methoxy-4-methansulfonyloxy-phenyl)-imidazo[4,5-e]-1,2,4-triazin

Hergestellt aus 0,75 g 2-(2-Methoxy-4-hydroxy- phenyl)-imidazo[4,5-e]-1,2,4-triazin und 1 ml Methansulfonsäurechlorid in einem Gemisch aus 25 ml Pyridin und 2 ml

2n Natronlauge, wobei 10 Stunden auf 50°C erwärmt wurde. Die Aufarbeitung erfolgt durch Abdampfen des Lösungsmittels, Versetzen des Rückstandes mit Eiswasser und Reinigen des ausgefallenen Rohprodukts über Kieselgel (Elutionsmittel: Methylenchlorid/Ethanol = 50:1 bis 25:1).
Ausbeute: 0,08 g (21 % der Theorie),
Schmelzpunkt: 217-219°C.

Beispiel 9

2-(2-Methoxy-4-cyanmethoxy-phenyl)-imidazo[4,5-e]-1,2,4-triazin

Hergestellt aus dem Kaliumsalz des 2-(2-Methoxy-4-hydroxy-phenyl)-imidazo[4,5-e]-1,2,4-triazin und Chloracetonitril.

Beispiel 10

2-(2-Methoxy-4-propargyloxy-phenyl)-imidazo[4,5-e]-1,2,4-triazin

Hergestellt analog Beispiel 9 aus dem Kaliumsalz des 2-(2-Methoxy-4-hydroxy-phenyl)-imidazo[4,5-e]-1,2,4-triazin und Propargylbromid.
Schmelzpunkt: 227-230°C

Beispiel 11

2-(2-Methoxy-4-methansulfonyloxy-phenyl)-imidazo[4,5-d]-pyridazin

Hergestellt analog Beispiel 3b aus 2-(2-Methoxy-4-hydroxy-phenyl)-imidazo[4,5-d]pyridazin.
Schmelzpunkt: 202-204°C (Zersetzung)

Beispiel 12

2-(2-Methoxy-4-methansulfonylamino-phenyl)-imidazo[4,5-d]-pyridazin

Hergestellt analog Beispiel 3b aus 2-(2-Methoxy-4-amino-phenyl)-imidazo[4,5-d]pyridazin.
Schmelzpunkt: 253-254°C (Zersetzung)

Beispiel 13

2-(2-Methoxy-4-methylsulfonyl-phenyl)-imidazo[4,5-d]pyridazin

Hergestellt durch Oxidation von 2-(2-Methoxy-4-methylmercapto-phenyl)-imidazo[4,5-d]pyridazin mit Wasserstoffperoxid in Eisessig.

Beispiel 14

2-(2-Methoxy-4-aminosulfonyl-phenyl)-imidazo[4,5-d]pyridazin

Hergestellt analog Beispiel 4b aus 2-(2-Methoxy-4-chlorsulfonyl-phenyl)-imidazo[4,5-d]pyridazin.
Schmelzpunkt: 228-230°C

Beispiel 15

2-(2-Methoxy-4-methylaminosulfonyl-phenyl)-imidazo[4,5-d]-pyridazin

Hergestellt analog Beispiel 4b aus 2-(2-Methoxy-4-chlorsulfonyl-phenyl)-imidazo[4,5-d]pyridazin und wäßriger Methylaminlösung.
Schmelzpunkt: 245-246°C

Beispiel 16

2-(2-Methoxy-4-dimethylaminosulfonyl-phenyl)-imidazo[4,5-d]-pyridazin

Hergestellt analog Beispiel 4b aus 2-(2-Methoxy-4-chlorsulfonyl-phenyl)-imidazo[4,5-d]pyridazin und wäßriger Dimethylaminlösung.
Schmelzpunkt: 219-220°C

Beispiel 17

2-(2-Methoxy-4-cyan-phenyl)-imidazo[4,5-d]pyridazin

Hergestellt aus 2-(2-Methoxy-4-amino-phenyl)-imidazo[4,5-d]-pyridazin durch Diazotieren und anschließende Umsetzung mit Kupfer(I)cyanid.

Beispiel 18

2-(2-Methoxy-4-aminocarbonyl-phenyl)-imidazo[4,5-d]pyridazin

Hergestellt aus 2-(2-Methoxy-4-cyan-phenyl)-imidazo[4,5-d]-pyridazin durch partielle Verseifung mit alkalischem Wasserstoffperoxid.

Beispiel 19

2-(2-Dimethylamino-4-nitro-phenyl)-imidazo[4,5-e]-1,2,4-triazin

Hergestellt analog Beispiel 2 aus 5,6-Diamino-1,2,4-triazin und 2-Dimethylamino-4-nitro-benzoesäure.

Beispiel 20

2-(2-Dimethylamino-4-amino-phenyl)-imidazo[4,5-e]-1,2,4-triazin

Hergestellt durch Reduktion von 2-(2-Dimethylamino-4-nitro-phenyl)-imidazo[4,5-e]-1,2,4-triazin mit Wasserstoff in Gegenwart von Palladiumkohle in Eisessig.

Beispiel 21

2-(2-Dimethylamino-4-acetylamino-phenyl)-imidazo[4,5-e]-1,2,4-triazin

Hergestellt aus 2-(2-Dimethylamino-4-amino-phenyl)-imidazo-[4,5-e]-1,2,4-triazin durch Erhitzen mit Acetanhydrid.

Beispiel 22

2-(2-Dimethylamino-4-methansulfonylamino-phenyl)-imidazo-[4,5-e]-1,2,4-triazin

Hergestellt analog Beispiel 3b aus 2-(2-Dimethylamino-4-amino-phenyl)-imidazo[4,5-e]-1,2,4-triazin.

Beispiel 23

2-(2-Methoxy-4-methylmercapto-phenyl)-imidazo[4,5-c]pyridazin

Hergestellt analog Beispiel 2 aus 3,4-Diamino-pyridazin und 2-Methoxy-4-methylmercapto-benzoesäure.
Schmelzpunkt: 190-192°C (Zersetzung)

Beispiel 24

2-(2-Methoxy-4-methylsulfinyl-phenyl)-imidazo[4,5-c]pyridazin

Hergestellt analog Beispiel 13 aus 2-(2-Methoxy-4-methylmercapto-phenyl)- imidazo[4,5-c]pyridazin.
Schmelzpunkt: 213-215°C (Zersetzung)

Beispiel 25

2-(2-Methoxy-4-methylsulfonyl-phenyl)-imidazo[4,5-c]pyridazin

Hergestellt analog Beispiel 13 aus 2-(2-Methoxy-4-methylmercapto-phenyl)-imidazo[4,5-c]pyridazin.
Schmelzpunkt: 196-198°C (Zersetzung)

Beispiel 26

2-(2-Methoxy-4-methansulfonyloxy-phenyl)-imidazo[4,5-c]pyridazin

Hergestellt aus 0,28 g 2-(2-Methoxy-4-hydroxy- phenyl)-imidazo[4,5-c]pyridazin und 0,52 g Methansulfonsäurechlorid in einer Lösung von 0,21 g Natriumhydroxid in 100 ml Wasser durch 2-tätiges Rühren bei Raumtemperatur.
Ausbeute: 0,2 g (55 % der Theorie),
Schmelzpunkt: 215-217°C (Zersetzung)

Beispiel 27

2-(2-Methoxy-4-chlor-phenyl)-imidazo[4,5-e]-1,2,4-triazin-hydrochlorid

Hergestellt aus 0,2 g 5,6-Diamino-1,2,4-triazin-hydrochlorid und 0,25 g 2-Methoxy-4-chlor-benzoesäure durch 3-stündiges Erhitzen auf 105-120°C in 25 g Polyphosphorsäure.
Ausbeute: 0,65 g (16 % der Theorie)
Schmelzpunkt: 220-222°C

Beispiel 28

2-(2-Methoxy-4-methylmercapto-phenyl)-imidazo[4,5-e]-1,2,4-triazin

Hergestellt analog Beispiel 2 aus 5,6-Diamino-1,2,4-triazin und 2-Methoxy-4-methylmercapto-benzoesäure.
Schmelzpunkt: 240-241°C

Beispiel 29

2-(2-Methoxy-4-methylsulfinyl-phenyl)-imidazo[4,5-e]-1,2,4-triazin

Hergestellt aus 2-(2-Methoxy-4-methylmercapto-phenyl)-imidazo[4,5-e]-1,2,4-triazin durch Oxidation mit Brom in Essigsäure in Gegenwart von Natriumacetat.

Beispiel 30

2-(2-Methoxy-4-methylsulfonyl-phenyl)-imidazo[4,5-e]-1,2,4-triazin

Hergestellt analog Beispiel 13 aus 2-(2-Methoxy-4-methylmercapto-phenyl)-imidazo[4,5-e]-1,2,4-triazin.
Schmelzpunkt: 261-264°C

Beispiel 31

2-(2-Methoxy-4-dimethylaminosulfonyl-phenyl)-imidazo[4,5-c]-pyridazin

Hergestellt analog Beispiel 27 aus 3,4-Diamino-pyridazin-hydrochlorid und 2-Methoxy-4-dimethylaminosulfonyl-benzoesäure.
Schmelzpunkt: 220-225°C

Beispiel 32

2-(2-Methoxy-4-benzyloxy-phenyl)-imidazo[4,5-c]pyridazin

Hergestellt analog Beispiel 2 aus 3,4-Diamino-pyridazin-hydrochlorid und 2-Methoxy-4-benzyloxy-benzoesäure.
Schmelzpunkt: 196-197°C

Beispiel 33

2-(2-Methoxy-4-amino-phenyl)-imidazo[4,5-d]pyridazin

Hergestellt analog Beispiel 27 aus 4,5-Diaminopyridazin und

2-Methoxy-4-amino-benzoesäure.
Schmelzpunkt: 270-271°C

Beispiel 34

2-(2-Methoxy-4-chlor-phenyl)-imidazo[4,5-d]pyridazin

Hergestellt aus 2-(2-Methoxy-4-amino-phenyl)-imidazo[4,5-d]-pyridazin durch Diazotieren in konzentrierter Salzsäure und Austausch der Diazoniumgruppe in Gegenwart von Kuper(I)-chlorid.
Schmelzpunkt: 260-262°C

Beispiel 35

2-(2-Methoxy-4-propargyloxy-phenyl)-imidazo[4,5-e]-1,2,4-triazin

Hergestellt analog Beispiel 2 aus 2-Methoxy-4-propargyloxy-benzoesäure und 5,6-Diamino-1,2,4-triazin.
Schmelzpunkt: 227-230°C

Beispiel 36

2-(2-Methoxy-4-aminocarbonyl-phenyl)-imidazo[4,5-c]pyridazin

Hergestellt analog Beispiel 27 aus 3,4-Diaminopyridazinhydrochlorid und 2-Methoxy-4-cyan-benzoesäure.
Schmelzpunkt: 275-285°C (Zersetzung), danach Wiederverfestigung und Schmelzen bei 315-323°C (Zersetzung)

Beispiel A

Tabletten zu 100 mg 2-(2-Methoxy-4-methansulfonyloxy-phenyl)-imidazo[4,5-b]pyrazin

Zusammensetzung
1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm
Trocknen: Umlufttrockenschrank 50°C
Trockensiebung: 1 mm
Dem Granulat die restlichen Hilfsstoffe zumischen und Endmischung zu Tabletten verpressen.
Tablettengewicht: 175 mg
Stempel: 8 mm

Beispiel B

Dragées zu 50 mg 2-(2-Methoxy-4-methansulfonyloxy-phenyl)-imidazo[4,5-b]pyrazin

Zusammensetzung:
1 Drageékern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

Feuchtsiebung: 1,0 mm
Trockensiebung: 1,0 mm,
Trocknung: 50°C im Umlufttrockenschrank

Granulat und restliche Hilfsstoffe mischen und zu Kernen verpressen.

Kerngewicht: 80 mg
Stempel: 6 mm
Wölbungsradius: 5 mm

Die fertigen Kerne werden auf übliche Weise mit einem Zuckerüberzug im Dragierkessel versehen.

Dragéegewicht: 120 mg

## Beispiel C

**Suppositorien zu 75 mg 2-(2-Methoxy-4-methansulfonyloxy-phenyl)-imidazo[4,5-b]pyrazin**

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

### Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 2-(2-Methoxy-4-methansulfonyloxyphenyl)-imidazo[4,5-b]pyrazin

1 Ampulle enthält:

| | |
|---|---|
| Wirksubstanz | 50,0 mg |
| Ethoxylierte Hydroxystearinsäure | 750,0 mg |
| 1,2-Propylenglykol | 1000,0 mg |
| Dest. Wasser ad | 5,0 ml |

Herstellungsverfahren:

Die Wirksubstanz wird in 1,2-Propylenglykol und ethoxylierter Hydroxystearinsäure gelöst, dann mit Wasser auf das angegebene Volumen aufgefüllt und steril filtriert.

Abfüllung: in Ampullen zu 5 ml

Sterilisation: 20 Minuten bei 120°C

Beispiel E

Tropfen zu 100 mg 2-(2-Methoxy-4-methansulfonyloxyphenyl)-imidazo[4,5-b]pyrazin

| | | |
|---|---|---|
| Wirksubstanz | | 1,0 g |
| p-Oxybenzoesäuremethylester | | 0,035 g |
| p-Oxybenzoesäurepropylester | | 0,015 g |
| Anisöl | | 0,05 g |
| Menthol | | 0,06 g |
| Saccharin-Natrium | | 1,0 g |
| Glycerin | | 10,0 g |
| Ethanol | | 40,0 g |
| Dest. Wasser | ad | 100,0 ml |

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

0148438

## Patentansprüche

1. Imidazolderivate der allgemeinen Formel

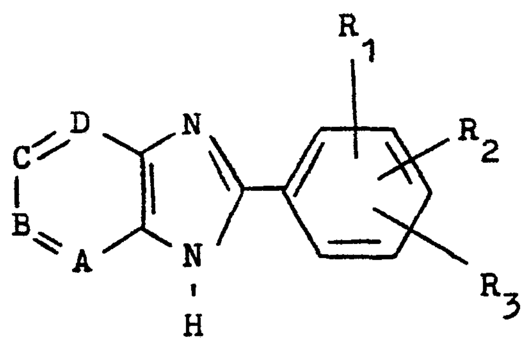

, (I)

in der

jeder der Reste A bis D ein N-Atom oder eine CH-Gruppe, wobei jedoch mindestens 2 der Reste A bis D N-Atome darstellen müssen und mit der Maßgabe, daß, wenn A und C gleichzeitig N-Atome bedeuten, auch B und/oder D je ein N-Atom darstellen muß,

$R_1$ ein Halogenatom, eine Alkyl-, Cyan-, Amino-, Alkylamino-, Benzyloxy-, Aminocarbonyl-, Alkylaminocarbonyl-, Dialkylaminocarbonyl-, Carboxy-, Alkoxycarbonyl-, Alkylsulfonyloxy-, Alkylsulfonyl-, Aminosulfonyl-, Alkylaminosulfonyl-, Dialkylaminosulfonyl-, Nitro-, Alkanoylamino-, Alkylsulfonylamino- oder N-Alkyl-alkylsulfonylaminogruppe oder auch, wenn 3 oder 4 der Reste A bis D je ein N-Atom und 0 oder 1 der Reste A bis D eine CH-Gruppe oder A und B je ein N-Atom und C und D je eine CH-Gruppe darstellen,

ein Wasserstoffatom, eine Hydroxy-, Alkoxy-, Phenylalkoxy-, Alkylmercapto-, Alkylsulfinyl-, Dialkylamino-, Cyanalkoxy-, Alkenyloxy- oder Alkinyloxygruppe,

$R_2$ und $R_3$, die gleich oder verschieden sein können, Wasserstoff- oder Halogenatome, Alkyl-, Hydroxy-, Alkoxy-, Phenylalkoxy-, Alkylmercapto-, Alkylsulfinyl-, Alkylsulfonyl-, Aminosulfonyl-, Alkylaminosulfonyl-, Dialkylaminosulfonyl-, Alkylsulfonyloxy-, Alkylsulfonylamino-, N-Alkyl-alkylsulfonylamino-, Nitro-, Amino-, Alkylamino-, Dialkylamino-, Cyan-alkoxy-, Cyan-, Carboxy-, Alkoxycarbonyl-, Aminocarbonyl-, Alkylaminocarbonyl-, Dialkylaminocarbonyl-, N-Alkanoyl-amino-, Alkenyloxy- oder Alkinyloxygruppe, wobei der Alkylteil jeweils 1 bis 3 Kohlenstoffatome und der Alkenyl- bzw. Alkinylteil jeweils 3 bis 5 Kohlenstoffatome enthalten kann, bedeuten, deren Tautomere und deren Säureadditionssalze.

2. Imidazolderivate der allgemeinen Formel I gemäß Anspruch 1, in der
3 oder 2 der Reste A bis D N-Atome und die übrigen der Reste A bis D CH-Gruppen darstellen, mit der Maßgabe, wenn A und C gleichzeitig N-Atome bedeuten, auch einer der Reste B und D ein N-Atom darstellt,

$R_1$ und $R_2$ ein Halogenatom, eine Methyl-, Methoxy-, Benzyloxy-, Ethoxy-, Propoxy-, Dimethylamino-, Amino-, Aminocarbonyl-, Aminosulfonyl-, Methylaminosulfonyl-, Dimethylaminosulfonyl-, Cyan-, Nitro-, Methylmercapto-, Methylsulfinyl-, Methylsulfonyl-, Methansulfonyloxy-, Methansulfonylamino-, N-Methyl-methansulfonylamino-, Propargyloxy- oder Cyanmethyloxygruppe und

$R_3$ ein Wasserstoffatom, eine Dimethylamino-, Methoxy-, Ethoxy-, Propoxy- oder Methylmercaptogruppe bedeuten, deren Tautomere und deren Säureadditionssalze.

3. Imidazolderivate der allgemeinen Formel I gemäß Anspruch 1, in der
$R_1$ eine Methyl-, Methoxy-, Methylmercapto-, Methylsulfinyl-, Methylsulfonyl-, Cyan-, Aminocarbonyl-, Methansulfonyloxy-, Aminosulfonyl-, Methylaminosulfonyl-, Methansulfonylamino- oder Dimethylaminosulfonylgruppe,
$R_2$ eine Methoxygruppe und
$R_3$ ein Wasserstoffatom bedeuten, deren Tautomere und deren Säureadditionssalze.

4. Imidazolderivate der allgemeinen Formel I gemäß Anspruch 1, in der
$R_1$ bis $R_3$ wie im Anspruch 3 definiert sind,
$R_1$ in 4- oder 5-Stellung und
$R_2$ in 2-Stellung des Phenylkerns stehen, deren Tautomeren und deren Säureadditionssalze.

5. 2-(2-Methoxy-4-methansulfonyloxy-phenyl)-imidazo[4,5-d]-pyridazin, dessen Tautomere und dessen Säureadditionssalze.

6. Physiologisch verträgliche Säureadditionssalze der Verbindungen gemäß den Ansprüchen 1-5.

7. Arzneimittel, enthaltend eine Verbindung gemäß den Ansprüchen 1-5 oder ein physiologisch verträgliches Säureadditionssalz gemäß Anspruch 6 neben einem oder mehreren inerten Trägerstoffen und/oder Verdünnungsmitteln.

8. Verwendung einer Verbindung gemäß den Ansprüchen 1-5 oder eines physiologisch verträglichen Säureadditionssalzes gemäß Anspruch 6 zur Behandlung von Herzinsuffizienzen.

9. Verfahren zur Herstellung eines Arzneimittels gemäß Anspruch 7, dadurch gekennzeichnet, daß auf nichtchemischem Wege eine Verbindung gemäß den Ansprüchen 1-5 oder ein physiologisch verträgliches Säureadditionssalz gemäß Anspruch

0148438

6 in einen oder mehreren inerten Trägerstoffen und/oder Verdünnungsmitteln eingearbeitet wird.

10. Verfahren zur Herstellung der Verbindungen gemäß den Ansprüchen 1-6 dadurch gekennzeichnet, daß

a) eine gegebenenfalls im Reaktionsgemisch hergestellte Verbindung der allgemeinen Formel

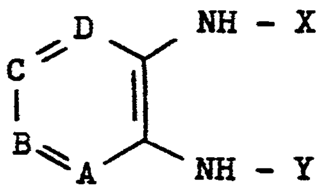

,(II)

in der
A bis D wie im Anspruch 1 definiert sind,
einer der Reste X oder Y ein Wasserstoffatom und der andere der beiden Reste X und Y oder beide Reste X und Y eine Gruppe der Formel

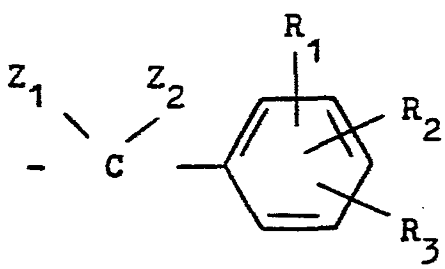

darstellen, in der

$R_1$ bis $R_3$ wie im Anspruch 1 definiert sind,
$Z_1$ und $Z_2$, die gleich oder verschieden sein können, Halogenatome, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen mono- oder disubstituierte Iminogruppe, eine Alkylendioxy- oder Alkylendithiogruppe mit jeweils 2 oder 3 Kohlenstoffatomen bedeuten, cyclisiert wird oder

b) zur Herstellung von Verbindungen der allgemeinen Formel I, in der mindestens einer der Reste $R_1$, $R_2$ oder $R_3$ eine Alkylsulfinyl- oder Alkylsulfonylgruppe darstellt, eine Verbindung der allgemeinen Formel

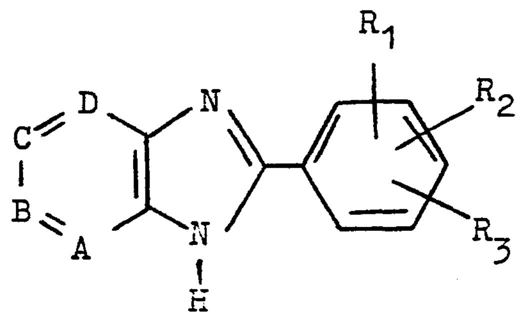

,(III)

in der
A bis D und $R_1$ bei $R_3$ wie im Anspruch 1 definiert sind, wobei
jedoch mindestens einer der Reste $R_1$, $R_2$ oder $R_3$ eine Alkylmercapto- oder Alkylsulfinylgruppe mit jeweils 1 bis 3 Kohlenstoffatomen darstellen muß, oxidiert wird oder

c) zur Herstellung von Verbindungen der allgemeinen Formel I, in der mindestens einer der Reste $R_1$, $R_2$ oder $R_3$ eine Alkylsulfonyloxy-, Alkylsulfonylamino-, N-Alkylalkylsulfonylamino- oder Alkanoylaminogruppe darstellt, eine Verbindung der allgemeinen Formel

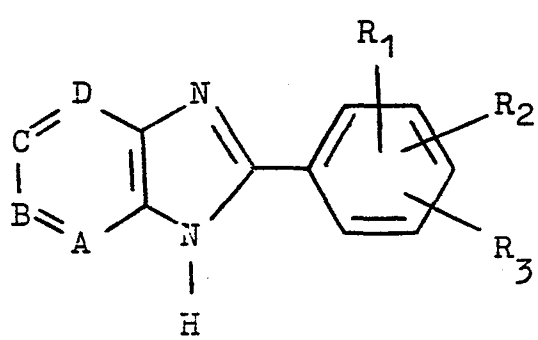

,(IV)

in der
A bis D und $R_1$ bis $R_3$ wie im Anspruch 1 definiert sind, wobei jedoch mindestens einer der Reste $R_1$, $R_2$ oder $R_3$ eine Hydroxy-, Amino- oder N-Alkylaminogruppe mit 1 bis 3 Kohlenstoffatomen im Alkylteil darstellen muß, mit einer Säure der allgemeinen Formel

$R_4$ - WOH ,(V)

in der
$R_4$ eine Alkylgruppe mit 1 bis 3 Kohlenstoffatomen und W eine Sulfonyl- oder Carbonylgruppe darstellen, in Gegenwart eines wasserentziehenden und/oder die Säure oder das Amin aktivierenden Mittels oder mit deren reaktionsfähigen Derivaten umgesetzt wird oder

d) zur Herstellung von Verbindungen der allgemeinen Formel I, in der mindestens einer der Reste $R_1$, $R_2$ oder $R_3$ eine durch eine Amino-, Alkylamino- oder Dialkylaminogruppe substituierte Carbonyl- oder Sulfonylgruppe darstellt, eine Verbindung der allgemeinen Formel

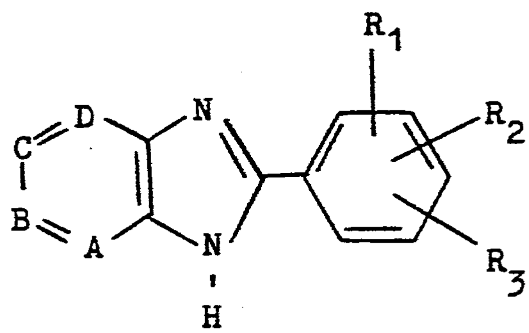
,(VI)

in der
A bis D und $R_1$ bis $R_3$ wie im Anspruch 1 definiert sind, wobei jedoch mindestens einer der Reste $R_1$, $R_2$ oder $R_3$ eine Carboxyl- oder Hydroxysulfonylgruppe darstellen muß, oder eines reaktionsfähigen Derivates hiervon mit einem Amin der allgemeinen Formel

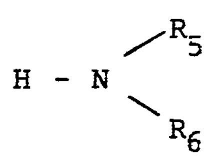
(VII)

in der
$R_5$ und $R_6$, die gleich oder verschieden sein können, Wasserstoffatome oder Alkylgruppen mit 1 bis 3 Kohlenstoffatome darstellen, oder mit einem reaktionsfähigen Derivat hiervon, falls mindestens einer der Reste $R_1$, $R_2$ oder $R_3$ die Carboxyl- oder Hydroxysulfonylgruppe darstellt, umgesetzt wird oder

e) zur Herstellung von Verbindungen der allgemeinen Formel I , in der mindestens einer der Reste $R_1$, $R_2$ oder $R_3$ eine Cyanalkoxy-, Alkenyloxy- oder Alkinyloxygruppe oder $R_2$ und/oder $R_3$ eine Alkoxygruppe darstellen, eine Verbindung der allgemeinen Formel

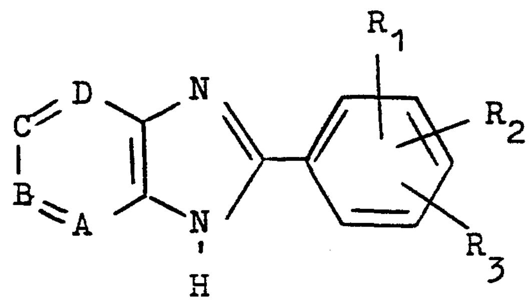

,(VIII)

in der
A bis D und $R_1$ bis $R_3$ wie eingangs definiert sind, wobei jedoch mindestes einer der Reste $R_1$, $R_2$ oder $R_3$ eine Hydroxygruppe darstellen muß, mit einer Verbindung der allgemeinen Formel

$$R_7 - U \quad ,(IX)$$

in der
$R_7$ eine gegebenenfalls durch eine Cyangruppe substituierte Alkylgruppe mit 1 bis 3 Kohlenstoffatomen, eine Alkenyl- oder Alkinylgruppe mit jeweils 3 bis 5 Kohlenstoffatomen und U eine nukleophile Austrittsgruppe wie ein Halogenatom, eine substituierte Sulfonyloxygruppe oder eine Alkoxysulfonyloxygruppe bedeuten, umgesetzt wird und

gewünschtenfalls anschließend eine so erhaltene Verbindung der allgemeinen Formel I, in der einer der Reste $R_1$, $R_2$ oder $R_3$ eine Benzyloxygruppe darstellt, mittels Entbenzylierung in die entsprechende Hydroxyverbindung übergeführt wird, und/oder

eine erhaltene Verbindung der allgemeinen Formel I, in der mindestens einer der Reste $R_1$, $R_2$ oder $R_3$ eine Cyangruppe darstellt, mittels Hydrolyse in die entsprechende Aminocarbonylverbindung übergeführt wird, und/oder

eine erhaltene Verbindung der allgemeinen Formel I, in der mindestens einer der Reste $R_2$ oder $R_3$ eine Nitrogruppe darstellt, mittels Reduktion in die entsprechende Aminoverbindung übergeführt wird, und/oder

eine erhaltene Verbindung der allgemeinen Formel I, in der einer der Reste $R_1$, $R_2$ oder $R_3$ eine Aminogruppe darstellt, über ihr Diazoniumsalz in eine entsprechende Verbindung der allgemeinen Formel I, in der einer der Reste $R_1$, $R_2$ oder $R_3$ ein Halogenatom, eine Hydroxy- oder Cyangruppe darstellt, übergeführt wird, und/oder

eine erhaltene Verbindung der allgemeinen Formel I in ihr Säureadditionssalz, insbesondere in ihr physiologisch verträgliches Säureadditionssalz mit einer anorganischen oder organischen Säure, übergeführt wird.